# EUROPEAN PATENT APPLICATION

(11) **EP 1 462 517 A1**
(43) Date of publication of application: **29.09.2004**
(21) Application number: 03701713.4
(22) Date of filing: 08.01.2003
(51) Int. Cl.: C12N 15/00, C12M 1/36, C12M 1/42, G01N 37/00, G01N 1/00, B25J 7/00, B01J 19/00, B01J 14/00, B81B 7/02

(54) **PCR METHOD BY ELECTROSTATIC TRANSPORTATION, HYBRIDIZATION METHOD FOR ELECTROSTATIC TRANSPORTATION AND DEVICES THEREFOR**

(30) Priority: 08.01.2002 JP 2002000972; 11.06.2002 JP 2002169461
(71) Applicant: Japan Science and Technology Agency, Kawaguchi-shi, Saitama 332-0012 (JP)
(72) Inventor: HIGUCHI, Toshiro, Yokohama-shi, Kanagawa 224-0006 (JP); TORII, Toru, Tokyo 167-0051 (JP); TANIGUCHI, Tomohiro, Funabashi-shi, Chiba 274-0063 (JP); KATAYAMA, Tetsuo, Tokyo 112-0005 (JP)
(74) Representative: Hoarton, Lloyd Douglas Charles
(86) International application number: PCT/JP2003/000049
(87) International publication number: WO 2003/057875

(57) **Abstract**

To provide a PCR method utilizing electrostatic transportation which allows separate control of individual DNA-containing droplets by accurately transporting the droplets, suitably controlling the temperature thereof and allowing a primer to react therewith; a hybridization method utilizing electrostatic transportation which allows rapid and easy detection of hybridization; and devices therefor.

A biological sample (droplets containing DNA and primer) (9) is prepared in a chemically inert liquid layer (8), is electrostatically transported by the action of an electrostatic transportation plate (1) with electrostatic transportation electrodes (2) and is heated at a predetermined position of the electrostatic transportation plate (1), thus carrying out PCR.

## Description

### Technical Field

The present invention relates to a method for carrying out polymerase chain reaction (PCR) utilizing electrostatic transportation, a method for carrying out hybridization utilizing electrostatic transportation and devices therefor.

### Background Art

Conventional techniques in this field can be found, for example, in the following documents.
(1) "Molecular Biology Illustrated", edited by Takaaki TAMURA and Tadashi YAMAMOTO, PP. 168-180, Yodosha Co., Ltd.
(2) "Biotechnological Experiments Illustrated", Hiroki NAKAYAMA, pp. 14-46, Shujunsha Co., Ltd.
(3) "Protocols for Molecular Biology", edited by Katsuro KOIKE, Takao SEKIYA and Hisato KONDO, PP. 217-224, Nankodo Co., Ltd.

Assay devices for post-genome applications which can be easily and reliably operated have been demanded in the field of biotechnology.

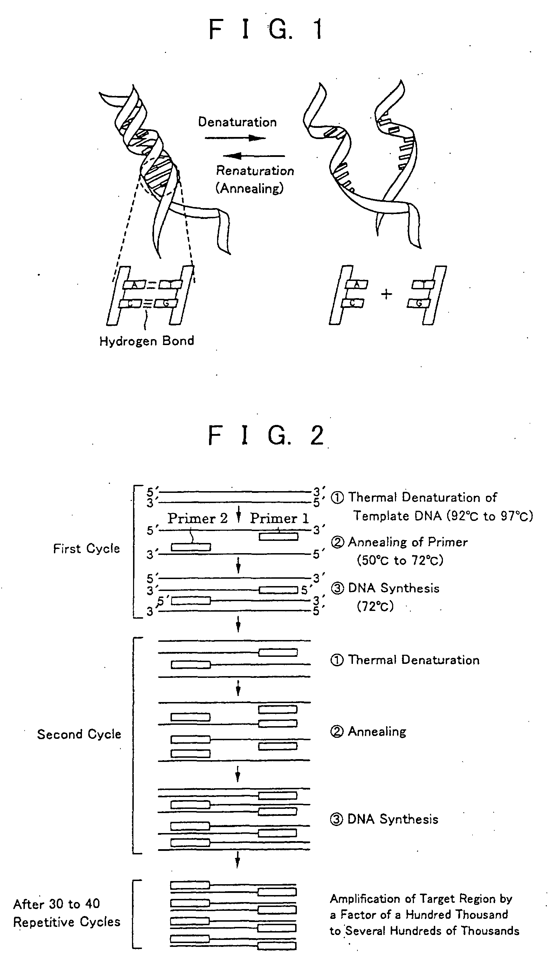

With reference to Fig. 1, a double helix DNA is denatured into single-stranded DNAs at high temperatures or under basic conditions because the hydrogen bonds constituting the double helix break. Single-stranded DNAs or RNAs having complementary base sequences are annealed under suitable conditions to form a double strand. This phenomenon is referred to as "hybridization".

There are a multitude of enzymes having different properties such as specificity which serve to synthesize, decompose or modify nucleic acids. Many genetic engineering techniques have been developed using properties of these enzymes. For example, a restriction enzyme process has played an essential role in DNA recombination technology for over twenty years. Heat-resistant DNA polymerases have been widely used in DNA amplification by using a polymerase chain reaction (PCR).

The PCR technique will be initially illustrated below.

In the PCR technique, a target DNA region is amplified from a trace amount of DNA by a factor of several tens of thousands in a short time.

The PCR technique is based on the principle shown in Fig. 2, in which a cycle comprising three steps of (1) thermally denaturing a template DNA, (2) annealing a primer to the template DNA, and (3)elongating the DNA strand using a heat-resistant DNA polymerase is repeated several tens of times. In recent years, steps (2) and (3) have been performed in one step in many cases. Namely, the PCR is performed in a cycle comprising two steps. Such a PCR technique is widely employed in various applications.

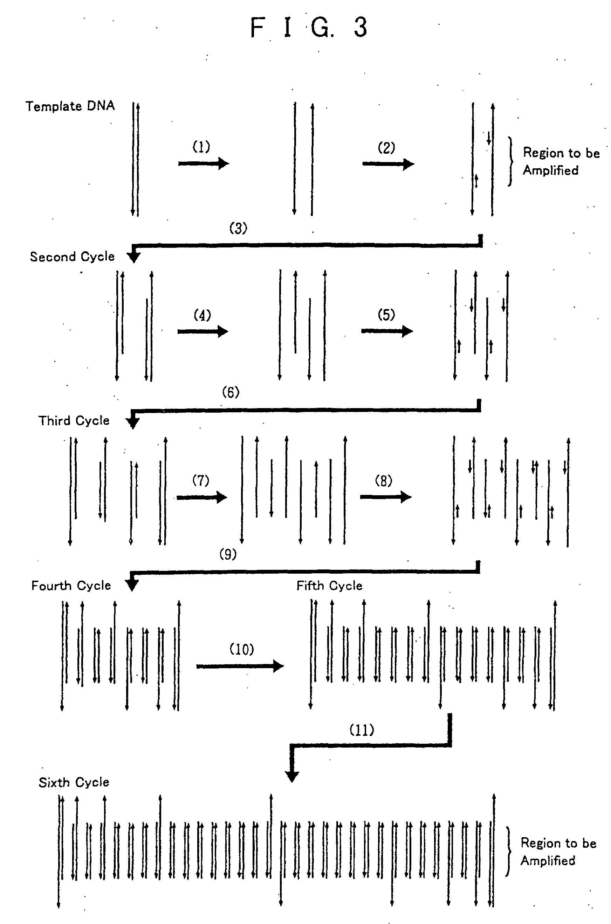

Fig. 3 shows the PCR technique in more detail.

A first cycle comprises (1) thermally denaturing a template DNA, (2) annealing a primer to the template DNA and (3) elongating the template DNA, and a second cycle comprises (4) thermally denaturing the template DNAs, (5) annealing a primer to the template DNAs and (6) elongating the template DNAs. A third cycle comprises (7) thermally denaturing the template DNAs, (8) annealing a primer to the template DNAs, and (9) elongating the template DNAs. After the three sequential cycles (10), a fourth cycle comprising thermal denaturation, annealing and elongation (11), and a fifth cycle comprising thermal denaturation, annealing and elongation are performed repetitively.

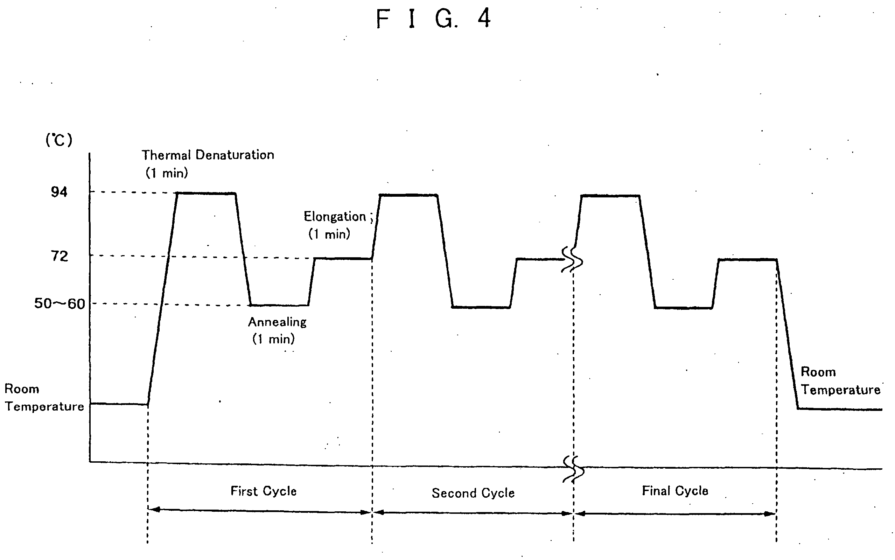

Fig. 4 shows illustrative standard temperatures and the time period for the PCR.

With reference to Fig. 4, temperature of a sample is raised from room temperature to 94°C to carry out thermal denaturation for about 1 minute, it is then decreased to a range from 50°C to 60°C to carry out annealing for about 1 minute, and then, it is raised to 72°C to carry out elongation for about 1 minute. This cycle is repeated sequentially.

Thus, the temperatures in multiple steps of PCR must be controlled precisely.

In most conventional hybridization methods, the hybridization is detected by mixing a known single-stranded DNA and an unknown single-stranded DNA sample in a DNA chip and observing the reaction therebetween.

### Disclosure of Invention

The position and temperature of a sample must be swiftly and properly controlled in the steps of PCR as described above. However, the conventional PCR processes require special micro-fluid devices such as microchannels, microvalves and micropumps and thus require complex operations and are difficult to perform smoothly.

Demands have therefore been made on assay devices for PCR that allow PCR to be carried out by easy and simple procedures.

Conventional DNA chips have a small contact area with a sample and thus exhibit a low detection sensitivity. As a possible solution to increase the contact area, for example, microbeads having a surface modified with a complementary deoxyribonucleic acid (cDNA) have been proposed. In addition, conventional hybridization methods are mainly performed manually, are thus complex, lack precision and take a long time to perform.

Under these circumstances, an object of the present invention is to provide a method for carrying out PCR utilizing electrostatic transportation, which can individually control DNA-containing droplets by precisely transporting the droplets, appropriately controlling the temperature of the droplets and treating the droplets with primers; a method for carrying out hybridization utilizing electrostatic transportation which ensures swift and easy detection; and devices therefor.

To achieve the above objects, the present invention provides:
[1] a method for carrying out PCR utilizing electrostatic transportation, comprising the steps of preparing a biological sample in a chemically inert liquid layer; electrostatically transporting the biological sample by the action of an electrostatic transportation plate with electrostatic transportation electrodes; and controlling the temperatures at predetermined positions of the electrostatic transportation plate and carrying out polymerase chain reaction;
[2] the method for carrying out PCR utilizing electrostatic transportation according to [1], wherein the biological sample comprises droplets containing DNA and primer;
[3] the method for carrying out PCR utilizing electrostatic transportation according to [1], wherein the biological sample comprises droplets containing DNA, and droplets containing primer;
[4] the method for carrying out PCR utilizing electrostatic transportation according to [1], further comprising arranging the electrodes in matrix form and electrostatically transporting the biological sample two-dimensionally;
[5] the method for carrying out PCR utilizing electrostatic transportation according to [1], further comprising applying voltage to heating electrodes and passing an electric current through the droplets to thereby heat the droplets;
[6] the method for carrying out PCR utilizing electrostatic transportation according to [5], wherein high-frequency alternating voltages are applied as the voltage;
[7] the method for carrying out PCR utilizing electrostatic transportation according to [1], further comprising maintaining the chemically inert liquid layer at a first temperature with thermostatic heater;
[8] the method for carrying out PCR utilizing electrostatic transportation according to [1], further comprising heating the biological sample in multiple steps;
[9] the method for carrying out PCR utilizing electrostatic transportation according to [1], further comprising arranging the electrodes and regions to be heated in a cascade and treating a plurality of the biological sample sequentially at specific time intervals;
[10] a device for carrying out PCR utilizing electrostatic transportation, comprising an electrostatic transportation plate with electrodes, the electrodes each having a water-repellent coating on a surface thereof; means for electrostatically transporting at least one biological sample by the action of the electrostatic transportation plate; and means for controlling the temperature of at least one biological sample at predetermined positions of the electrostatic transportation plate;
[11] the.device for carrying out PCR utilizing electrostatic transportation according to [10], wherein the electrodes are arranged in matrix form;
[12] the device for carrying out PCR utilizing electrostatic transportation according to [10], further comprising a sheet-like heater layer for thermostatically heating the chemically inert liquid layer;
[13] the device for carrying out PCR utilizing electrostatic transportation according to [10], wherein the means for heating the biological sample is a heating plate for applying voltage to droplets;
[14] the device for carrying out PCR utilizing electrostatic transportation according to [13], wherein the voltage is a high-frequency alternating voltage;
[15] the device for carrying out PCR utilizing electrostatic transportation according to [10], wherein the means for heating the biological sample is a long heater layer;
[16]. a method for carrying out PCR utilizing electrostatic transportation, comprising the steps of preparing droplets containing DNA and primer in a chemically inert liquid layer; electrostatically transporting the droplets containing DNA and primer by the action of an electrostatic transportation plate with electrostatic transportation electrodes; heating and thermally denaturing the DNA at a first position of the electrostatic transportation plate; annealing primer to the DNA, the primer being capable of reacting with the DNA; and elongating the annealed DNA;
[17] a device for carrying out PCR utilizing electrostatic transportation, comprising an electrostatic transportation plate with electrodes, the electrodes each having a water-repellent coating on a surface thereof; a chemically inert liquid layer carrying droplets containing DNA and primer; means for heating the droplets containing DNA and primer at predetermined positions; means for electrostatically transporting the droplets containing DNA and primer by the action of the electrostatic transportation plate; means for heating and thermally denaturing the DNA at a first position of the electrostatic transportation plate; means for annealing primer to the DNA, the primer being capable of reacting with the DNA; and means for elongating the annealed DNA;
[18] the device for carrying out PCR utilizing electrostatic transportation according to [17], wherein the electrodes are arranged in matrix form;
[19] the method for carrying out PCR utilizing electrostatic transportation according to [1] or [16], further comprising detecting and controlling the position of the biological sample;
[20] the device for carrying out PCR utilizing electrostatic transportation according to [10] or [17], further comprising means including a computer, an image pickup device being connected to the computer, and a controller being connected to the computer, wherein the means is so configured as to monitor the motion of the biological sample with the image pickup device and to control the position of the biological sample by the controller through the computer based on the monitoring;
[21] a method for carrying out hybridization utilizing electrostatic transportation, comprising the steps of covering an electrostatic transportation electrode substrate with a chemically inert liquid; arranging an array of droplets containing respective known single-stranded DNAs and fluorescence reagent therein; electrostatically transporting droplets containing an unknown single-stranded DNA sample; combining the sample droplets with the respective droplets containing the known single-stranded DNAs and the fluorescence reagent to thereby carry out hybridization; and detecting the hybridization based on the fact that the fluorescence reagent is intercalated between the known and unknown single-stranded DNAs when the two DNAs match with each other and fluorescence is emitted at a varying intensity depending on the degree of match between the two DNAs; and
[22] a device for carrying out hybridization utilizing electrostatic transportation, comprising an electrostatic transportation electrode substrate being filled with a chemically inert liquid layer; droplets being arranged in an array on the electrostatic transportation electrode substrate and each comprising known single-stranded DNA and fluorescence reagent; droplets containing unknown single-stranded DNA sample to be transported by the action of electrodes of the electrostatic transportation electrode substrate; and means for treating the droplets containing the unknown single-stranded DNA sample with the respective droplets containing the known single-stranded DNAs and the fluorescence reagent and detecting the hybridization between the two DNAs.

The term "known single-stranded DNA" as used herein includes cDNA.

### Brief Description of the Drawings

Fig. 1 schematically illustrates denaturation and renaturation of a double-stranded DNA.
Fig. 2 schematically illustrates the principle of PCR.
Fig. 3 is a flow chart of PCR.
Fig. 4 is a profile of the temperature and time period in PCR.
Fig. 5 schematically illustrates a PCR device (chip for PCR) utilizing electrostatic transportation according to a first embodiment of the present invention.
Fig. 6 schematically illustrates an electrostatic transportation plate and transportation of droplets containing DNA as a biological sample and a primer, in which heating means is not shown, according to the first embodiment of the present invention.
Fig. 7 is a schematic diagram of the entire PCR device according to the first embodiment of the present invention.
Fig. 8 is a sectional view of a cell in the PCR device according to the first embodiment of the present invention.
Fig. 9 schematically illustrates a heating device for droplets according to the first embodiment of the present invention.
Fig. 10 schematically illustrates a modification of the heating device for a biological sample according to the first embodiment of the present invention.
Fig. 11 schematically illustrates PCR according to another embodiment of the present invention.
Fig. 12 schematically illustrates a PCR device utilizing electrostatic transportation according to a second embodiment of the present invention.
Fig. 13 schematically illustrates an electrostatic transportation plate according to the second embodiment of the present invention and transportation of droplets containing DNA as a biological sample and primer, in which heating means is not shown.
Fig. 14 is a schematic diagram of the PCR device according to the second embodiment of the present invention.
Fig. 15 is a sectional view of a cell in a PCR device according to a third embodiment of the present invention.
Fig. 16 is a sectional view of a cell in a PCR device according to a fourth embodiment of the present invention.
Fig. 17 is a sectional view of a cell in a PCR device according to a fifth embodiment of the present invention.
Fig. 18 is a sectional view of a cell in a PCR device according to a sixth embodiment of the present invention.
Fig. 19 is a sectional view of a cell in a PCR device according to a seventh embodiment of the present invention.
Fig. 20 is a sectional view of a cell in a PCR device according to an eighth embodiment of the present invention.
Fig. 21 is a sectional view of a cell in a PCR device according to a ninth embodiment of the present invention.
Fig. 22 is a schematic diagram of a device for carrying out hybridization using electrostatic transportation as yet another embodiment of the present invention.
Fig. 23 schematically illustrates the emission of fluorescence.
Fig. 24 is a diagram showing fluorescence intensities observed in the hybridization device utilizing electrostatic transportation as yet another embodiment of the present invention.
Fig. 25 is a schematic diagram of an image pickup device for determining the intensity of fluorescence emitted as a result of hybridization in Fig. 24.
Fig. 26 is a schematic diagram of an image pickup device for determining the intensity of fluorescence emitted as a result of hybridization according to yet another embodiment of the present invention.

### Best Mode for Carrying Out the Invention

Certain embodiments of the present invention will be illustrated in detail below.

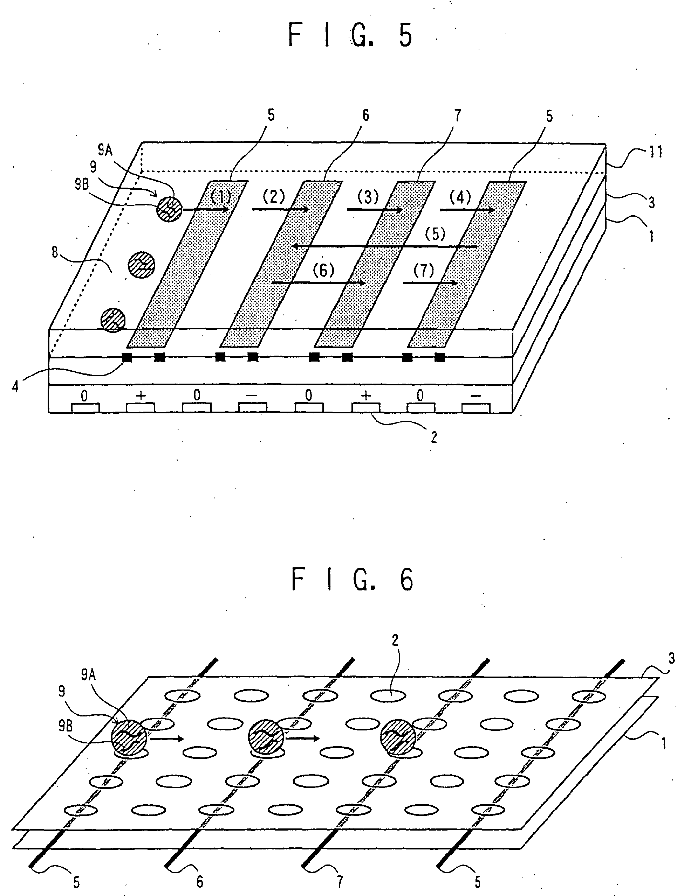

Figs. 5, 6, 7, 8 and 9 are schematic diagrams of a PCR device (chip for PCR) utilizing electrostatic transportation, an electrostatic transportation plate of the device and transportation of droplets containing DNA as a biological sample and primer (heating means is not shown), the entire PCR device, a cell in section of the PCR device, a heating element for droplets of the device, respectively, according to the first embodiment of the present invention.

These figures illustrate an electrostatic transportation plate 1 with electrostatic transportation electrodes (dot electrodes) 2 arranged in matrix form; a heating plate 3 arranged on the plate 1 and having electrodes 4 for heating; a first heating region 5; a second. heating region 6; a third heating region 7; a chemically inert liquid layer 8; a biological sample 9 which comprises droplets containing DNA 9A and primer 9B herein; a cell 11; a digital signal processing (DSP) controller 12; a programmable electric source 13; a personal computer 14; and a video camera 15.

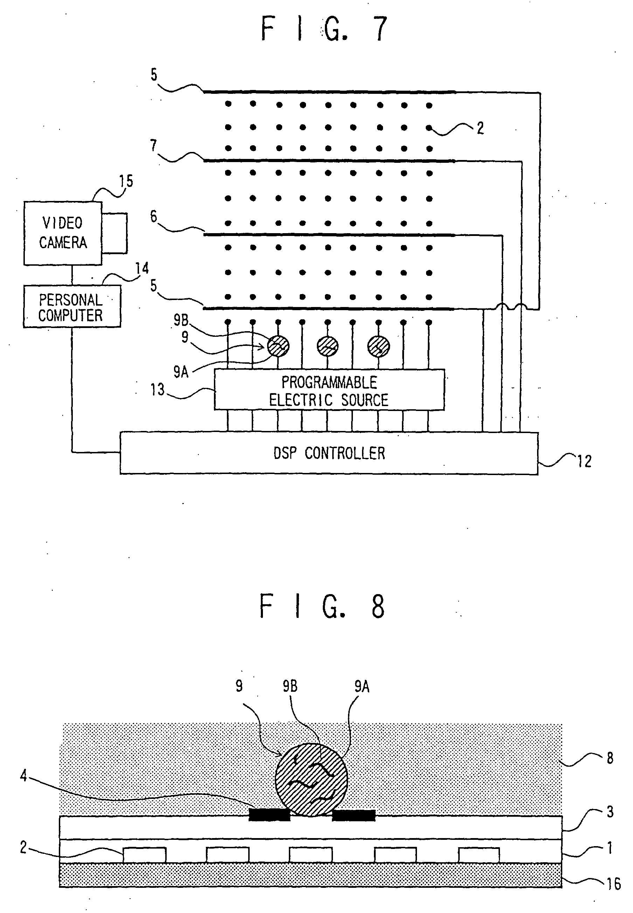

Thus, for example, the biological sample (droplets containing DNA and primer) 9 is prepared in the chemically inert liquid layer 8 and is subjected to repetitive cycles of thermal denaturation, annealing and elongation steps. In this embodiment, the heating plate 3 with heating electrodes 4 thereon is arranged on the electrostatic transportation plate 1 with the electrostatic transportation electrodes (dot electrodes) 2 arranged in matrix form. The cell 11 carries the chemically inert liquid layer 8 containing the biological sample (droplets containing DNA and primer) 9 and is arranged on the heating plate 3. The device shown in Fig. 8 further comprises a heater layer 16 at the bottom thereof. The heater layer 16 serves as a thermostatic heater for controlling the temperature of atmosphere of the cell 11.

The biological sample (droplets containing DNA and primer) 9 can be handled or moved two-dimensionally in an arbitrary direction by placing the biological sample (droplets containing DNA and primer) 9 above the electrostatic transportation plate 1 with electrostatic transportation electrodes (dot electrodes) 2 arranged two-dimensionally and controlling the voltage applied to the electrostatic transportation electrodes (dot electrodes) 2 by the DSP controller 12 connected to the personal computer 14.

The biological sample (droplets containing DNA and primer) 9 moves because the surface thereof is positively or negatively charged, which causes attraction or repulsion with the electrostatic transportation electrodes (dot electrodes) 2. By applying a voltage in the form of a traveling wave to the electrostatic transportation electrodes (dot electrodes) 2, the electrostatic transportation electrodes (dot electrodes) 2 provide a driving force to the biological sample (droplets containing DNA and primer) 9. In addition, the two-dimensional arrangement of the electrostatic transportation electrodes (dot electrodes) 2 allows the biological sample (droplets containing DNA and primer) 9 to be handled or moved two-dimensionally in an arbitrary direction.

The motion of the biological sample is monitored, for example, by the video camera (image pickup device) 15 connected to the personal computer 14. Based on the monitoring, the DSP controller 12 and the programmable electric source 13 are controlled through the personal computer 14 to thereby control the position of the biological sample (droplets containing DNA and primer) 9.

The electrostatic transportation electrodes (dot electrodes) 2 may be arranged in lines in parallel with the X or Y axis or may be in the form of dots in which points of intersection alone serve as electrodes.

The biological sample (droplets containing DNA and primer) 9 is then transported by the action of the voltage applied to electrostatic transportation electrodes (dot electrodes) 2 of the electrostatic transportation plate 1. The first heating region 5 is arranged on the transportation path to thereby activate or thermally denature the biological sample (droplets containing DNA and primer) 9. Electrostatic transportation of micro-droplets has been proposed by the present inventors in Japanese Patent Application No. 2001-238625.

Thus, with reference to Fig. 5, the biological sample (droplets containing DNA and primer) 9 is transported by the action of the voltage applied to the electrostatic transportation electrodes (dot electrodes) 2, is heated in the first heating region 5 at about 92°C to 97°C in a short time (on the order of seconds)-and is thereby thermally denatured [Step (1) in Fig. 3].

Then the biological sample (droplets containing DNA and primer) 9 is transported to the second heating region 6, is heated therein at about 50°C to 72°C in a short time (on the order of seconds), and thereby the primer 9B is annealed [Step (2)].

After the annealing, the biological sample (droplets containing DNA and primer) 9 is transported to the third heating region 7 by the action of the voltage applied to the electrostatic transportation electrodes (dot electrodes) 2, is heated therein at about 72°C in a short time (on the order of seconds), and thereby the DNA 9A is elongated [Step (3)]. PCR can be performed by repeating these steps about 25 times. More specifically, after the elongation step [Step (3)], the biological sample (droplets containing DNA and primer) 9 is moved downstream therefrom, is heated again in the heating region 5 at about 92°C to 97°C in a short time and is thereby thermally denatured [Step (4)], is then moved to the heating region 6, is annealed therein [Step (5)], is moved to the heating region 7 and is elongated [Step (6)]. Then, the subsequent thermal denaturation step [Step (7)] is performed. PCR is performed by repeating these steps for a predetermined number of cycles. In practice, these steps are repeated about 25 times. The configurations of Steps (1) to (7) are not specifically limited, and various modifications can be employed.

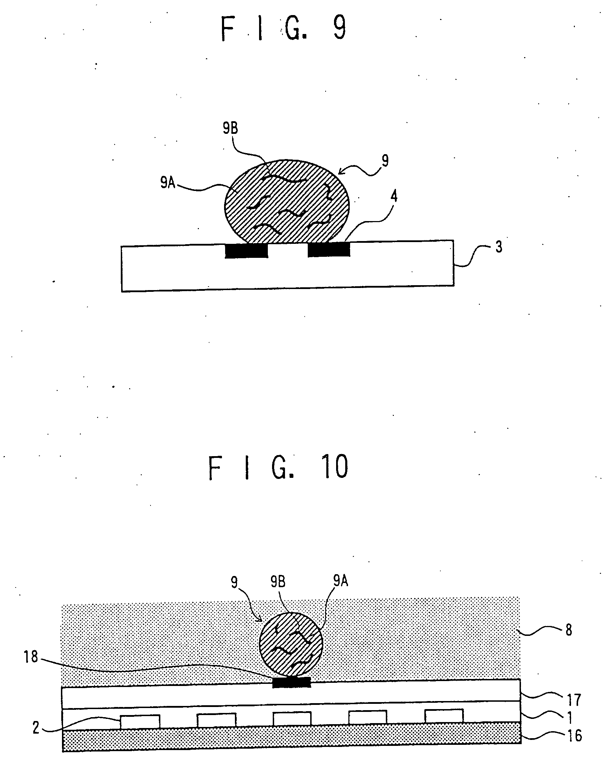

Figs. 8 and 9 each shows a device for heating the biological sample used in this embodiment. The biological sample (droplets containing DNA and primer) 9 to be heated is located on the heating electrodes 4 on the heating plate 3 by the action of the electrostatic transportation electrodes (dot electrodes) 2. Then, a high-frequency alternating voltage is applied to the heating electrode 4 to thereby heat the biological sample (droplets containing DNA and primer) 9 directly. Thus, thermal denaturation, annealing and elongation steps are performed.

A heating device shown in Fig. 10 can also be used as the heating device for heating the biological sample (droplets containing DNA and primer) 9. In this heating device, a long heater layer 18 is arranged on a surface of a heat-insulative substrate 17. An electric source is connected to the long heater layer 18 and applies electric current thereto to thereby heat the long heater layer 18. The biological sample (droplets containing DNA and primer) 9 is transported and is located on the long heater layer 18 by the action of the electrostatic transportation plate 1 with the electrostatic transportation electrodes (dot electrodes) 2 and is then heated by the long heater layer 18. Thus, thermal denaturation, annealing and elongation are performed

A method for producing such droplets has been proposed by the present inventors in Japanese Patent Application No. 2001-238624.

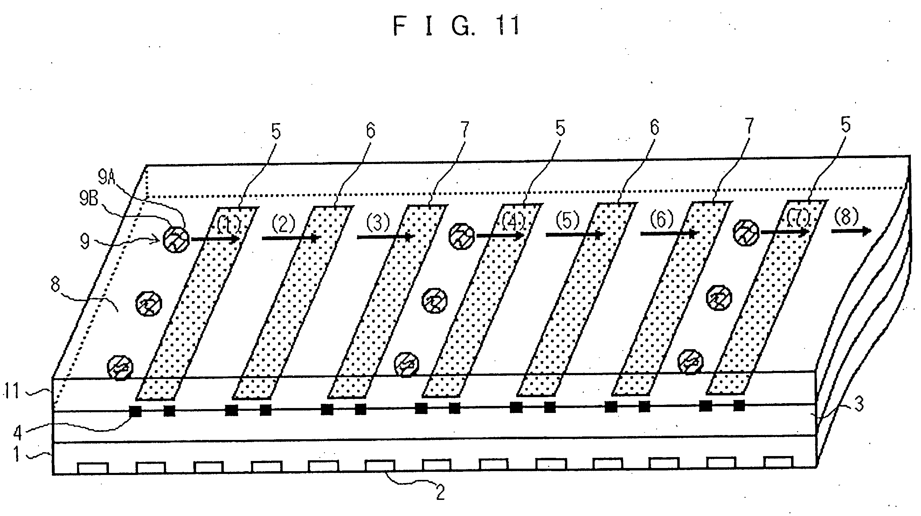

Fig. 11 schematically illustrates yet another embodiment of PCR according to the present invention.

In this embodiment, plural sets of electrodes and heating regions are arranged sequentially along the path that the biological sample (droplets containing DNA and primer) 9 moves. Plural PCR cycles can thereby be performed sequentially at certain time intervals.

Thus, a large multiplicity of PCR can be efficiently performed sequentially in a flow system, by feeding a plurality of the biological sample (droplets containing DNA and primer) 9 sequentially.

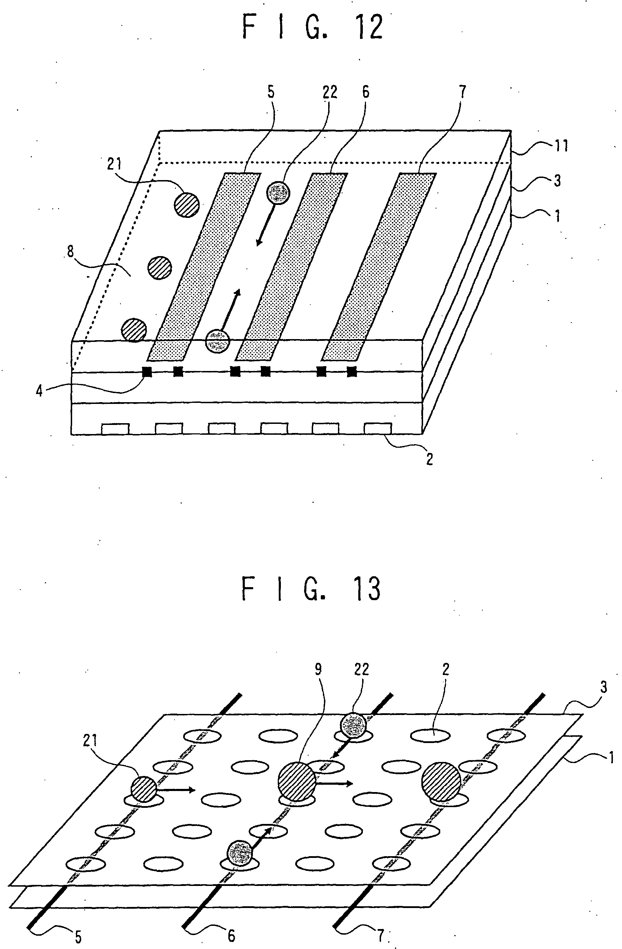

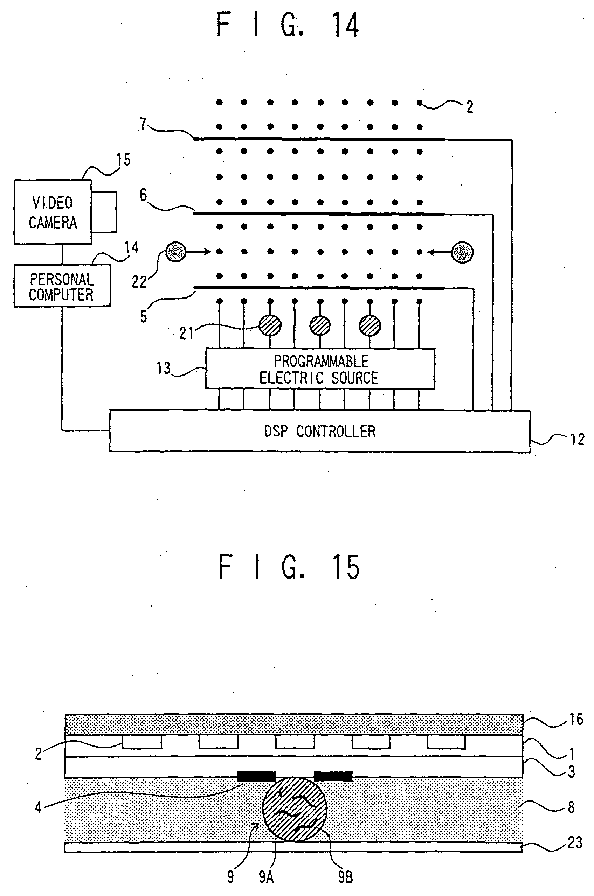

Figs. 12, 13 and 14 are schematic diagrams of a PCR device utilizing electrostatic transportation, an electrostatic transportation plate and transportation of droplets containing DNA as a biological sample and primer (heating means is not shown), and the entire PCR device, respectively, according to a second embodiment of the present invention. The same components as in the first embodiment are given with the same reference numerals, and a detailed description thereof will be omitted.

Figs. 12 to 14 illustrate a biological sample (DNA-containing droplets) 21 and primer-containing droplets 22.

Thus, for example, the biological sample (DNA-containing droplets) 21 is prepared in the chemically inert liquid layer 8 and is subjected to repetitive cycles of thermal denaturation, annealing and elongation steps while allowing the primer-containing droplets 22 to act thereon. In this embodiment, the heating plate 3 with heating electrodes 4 thereon is arranged on an electrostatic transportation plate 1 with electrostatic transportation electrodes (dot electrodes) 2 arranged in matrix form. The cell 11 houses a chemically inert liquid layer 8 carrying the biological sample (DNA-containing droplets) 21 and the primer-containing droplets 22 and is arranged on the heating plate 3.

The biological sample (DNA-containing droplets) 21 is transported by the action of the voltage applied to the electrostatic transportation electrodes (dot electrodes) 2 of the electrostatic transportation plate 1. A first heating region 5 is arranged on the transportation path to thereby heat and thermally denature the biological sample (DNA-containing droplets) 21.

The primer-containing droplets 22 are also transported by the action of the voltage applied to the electrostatic transportation electrodes (dot electrodes) 2 of the electrostatic transportation plate 1. Thus, the primer-containing droplets 22 are combined with the biological sample (DNA-containing droplets) 21 and thereby react therewith.

Thus, the primer-containing droplets 22 reacted with the biological sample (DNA-containing droplet) 21 are transported by the action of.the voltage applied to the electrostatic transportation electrodes (dot electrodes) 2, are heated in a second heating region 6 and are thereby annealed.

After the annealing, the combined droplets are transported to the third heating region 7 by the action of the voltage applied to the electrostatic transportation electrodes (dot electrodes) 2 and the DNA is then elongated. Thus, PCR is performed. In practice, these steps are repeated about 25 times.

Any of the heating devices shown in Figs. 8 to 10 can be used in this embodiment for heating the droplets.

Configurational embodiments of cells used in the PCR devices according to the present invention will be illustrated below.

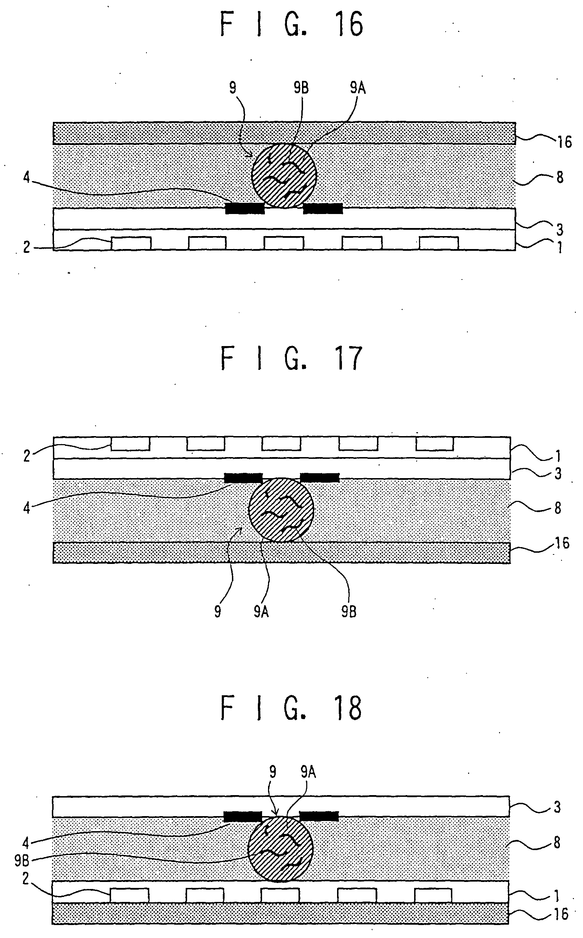

Fig. 15 is a sectional view of a cell in a PCR device according to a third embodiment of the present invention.

In this embodiment, only a bottom plate 23 is arranged under a chemically inert liquid layer 8 carrying a biological sample (droplets containing DNA 9A and primer 9B) 9. A heating plate 3 with heating electrodes 4, an electrostatic transportation plate 1 with electrostatic transportation electrodes (dot electrodes) 2, and a thermostatic heater layer 16 are sequentially arranged in this order on or above the chemically inert liquid layer 8.

Fig. 16 is a sectional view of a cell in a PCR device according to a fourth embodiment of the present invention.

In this embodiment, an electrostatic transportation plate 1 with electrostatic transportation electrodes (dot electrodes) 2, a heating plate 3 with heating electrodes 4, and a chemically inert liquid layer 8 carrying a biological sample (droplets containing DNA 9A and primer 9B) 9 are arranged in this order from the bottom. Only a thermostatic heater layer 16 is arranged above the chemically inert liquid layer 8.

Fig. 17 is a sectional view of a cell in a PCR device according to a fifth embodiment of the present invention.

In this embodiment, only a thermostatic heater layer 16 is arranged under a chemically inert liquid layer 8 containing a biological sample (droplets containing DNA 9A and primer 9B) 9. Thereabove, a heating plate 3 with heating electrodes 4 and an electrostatic transportation plate 1 with electrostatic transportation electrodes (dot electrodes) 2 are sequentially arranged.

Fig. 18 is a sectional view of a cell in a PCR device according to a sixth embodiment of the present invention.

In this embodiment, a thermostatic heater layer 16, a electrostatic transportation plate 1 with electrostatic transportation electrodes (dot electrodes) 2, and a chemically inert liquid layer 8 carrying a biological sample (droplets containing DNA 9A and primer 9B) 9 are arranged in this order from the bottom. Only a heating plate 3 with heating electrodes 4 is arranged above the chemically inert liquid layer 8.

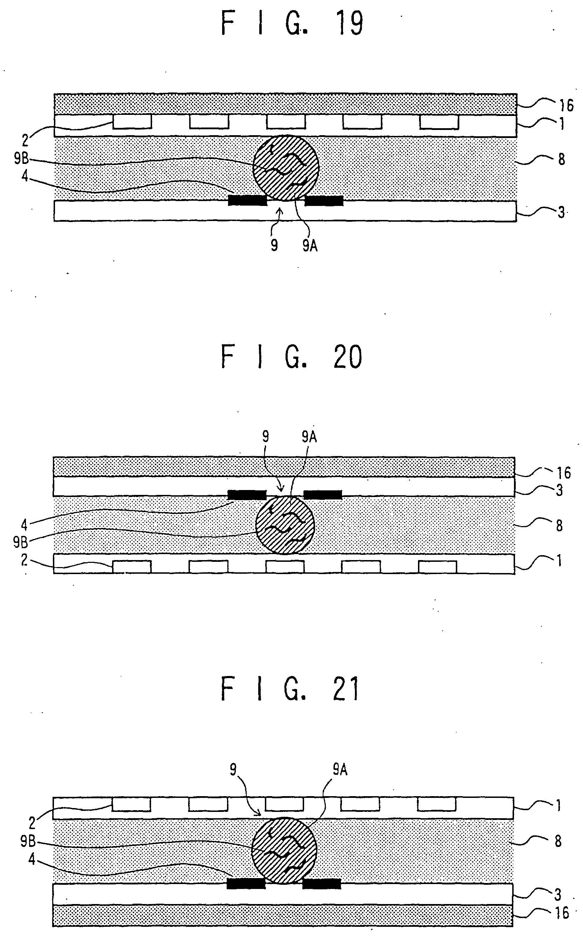

Fig. 19 is a sectional view of a cell in a PCR device according to a seventh embodiment of the present invention.

In this embodiment, only a heating plate 3 with heating electrodes 4 is arranged below a chemically inert liquid layer 8 carrying a biological sample (droplets containing DNA 9A and primer 9B) 9. Thereabove, an electrostatic transportation plate 1 with electrostatic transportation electrodes (dot electrodes) 2 and a thermostatic heater layer 16 are sequentially arranged in this order.

Fig. 20 is a sectional view of a cell in a PCR device according to an eighth embodiment of the present invention.

In this embodiment, only an electrostatic transportation plate 1 with electrostatic transportation electrodes (dot electrodes) 2 is arranged below a chemically inert liquid layer 8 carrying a biological sample (droplets containing DNA 9A and primer 9B) 9. Thereabove, a heating plate 3 with heating electrodes 4 and a thermostatic heater layer 16 are sequentially arranged in this order.

Fig. 21 is a sectional view of a cell in a PCR device according to a ninth embodiment of the present invention.

In this embodiment, a thermostatic heater layer 16, a heating plate 3 with heating electrodes 4, and a chemically inert liquid layer 8 carrying a biological sample (droplets containing DNA 9A and primer 9B) 9 are sequentially arranged in this order from the bottom. Only an electrostatic transportation plate 1 with electrostatic transportation electrodes (dot electrodes) 2 is arranged above the chemically inert liquid layer 8.

A long heater layer as shown in Fig. 10 can be used instead of the heating plate 3 with the heating electrodes 4 in the above embodiments. It may be arranged in a direction perpendicular to the moving direction of the droplet. Such a long heater layer 18 (not shown) can be arranged instead of any of the heating plates 3 with the heating electrodes 4 shown in Figs. 15 through 21.

The above-exemplified electrostatic transportation electrodes (dot electrodes) are arranged in matrix form. However, they can also be arranged in lines.

In addition, the droplets can be handled one-dimensionally instead of two-dimensionally.

Thus, the present invention provides assay chip devices ("Lab on a chip" devices) for PCR. More specifically, the present invention provides a method for carrying out PCR and a device (chip device) therefor. In this system, electrodes are arranged in matrix form; a chip housing a chemically inert liquid layer is prepared as a reaction field; plural small DNA-containing droplets as a biological sample and plural primer-containing droplets are fed thereto; a voltage is applied to the electrodes or a heater layer is energized; thus the DNA-containing droplets or primer-containing droplets are arbitrarily moved, are combined with each other by the action of electrostatic force and are reacted by heating simultaneously or sequentially.

The application of a voltage to the electrodes can also be used to elevate the temperature of the droplets. This PCR system does not require pumps and channels and can amplify a plurality of different DNAs simultaneously on one chip even with trace amounts of reagents.

The present invention develops and provides a chip for carrying out PCR in which droplets of a DNA sample as a biological sample in an inert liquid are combined with droplets containing a plurality of different primers. Thus, a plurality of different DNAs can be amplified simultaneously on one chip.

The present invention thus configured exhibits the following advantages.
(1) Micro-droplets contained in a chemically inert liquid layer are used, and only trace amounts of the sample and reagents are required.
(2) The droplets are transported by an electrostatic force and can be moved two-dimensionally on electrodes. The PCR system thereby does not require special micro-fluid devices such as microchannels, microvalves and micropumps.
(3) Multiple chemical reactions can be performed simultaneously or sequentially on a substrate.
(4) The micro-droplets can be heated to a suitable temperature at a predetermined position by utilizing the electric conductivity of the micro-droplets and passing an electric current therethrough.
(5) By using a long heater for heating the micro-droplets, the heating device can be simplified and produced at lower cost.
(6) The temperature of the cell on the chip can be set at a predetermined temperature by using a thermostatic heater.
(7) The position and temperature of the droplets can be accurately controlled, and the droplets can be handled easily and precisely.

Yet another embodiment of the present invention will be illustrated below.

According to this embodiment, a hybridization method and a device therefor utilizing electrostatic transportation are provided. In this system, known single-stranded DNAs are held on an electrostatic transportation substrate filled with a liquid, an unknown single-stranded DNA sample is transported and reacted with the known single-stranded DNAs by the action of an electrostatic force, and the hybridization is detected based on the emitted light.

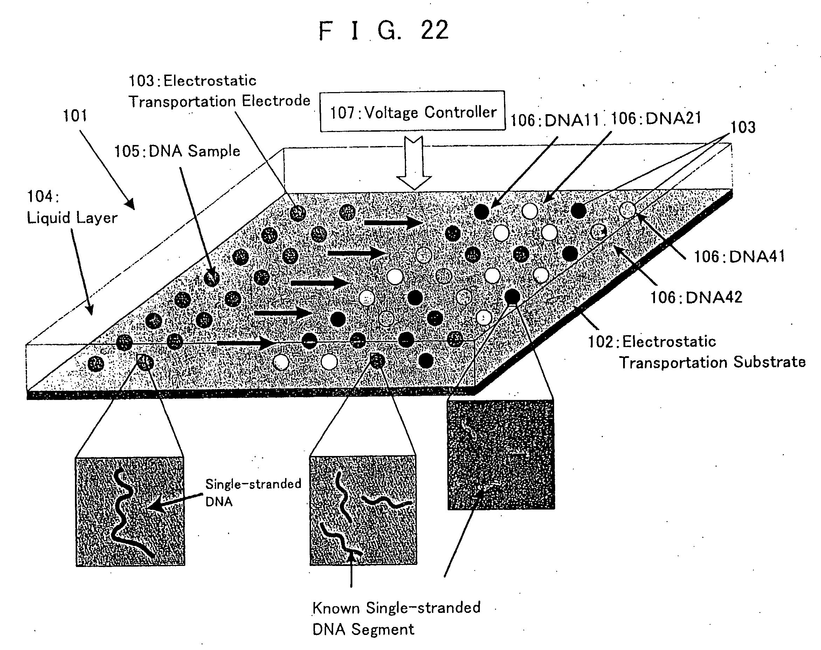

Fig. 22 is a schematic diagram of a hybridization device utilizing electrostatic transportation as yet another embodiment of the present invention.

Fig. 22 shows the hybridization device utilizing electrostatic transportation 101, an electrostatic transportation electrode substrate 102, electrostatic transportation electrodes 103, a liquid layer 104, droplets 105 each containing an unknown single-stranded DNA sample, droplets 106 containing single-stranded DNAs having known base sequences [DNA 11, DNA 12, ... DNA 41, DNA 42...] and a fluorescence reagent, and a voltage controller 107. The voltage controller 107 is connected to the electrostatic transportation electrodes 103 spread over the electrostatic transportation electrode substrate 102.

The droplets 106 each containing single-stranded DNA having a known base sequence and a fluorescence reagent are arranged.in an array on the electrostatic transportation electrode substrate 102. The droplets 105 containing an unknown single-stranded DNA are moved utilizing electrostatic transportation and are combined with.the droplets 106 each containing single-stranded DNA having a known base sequence and a fluorescence reagent.

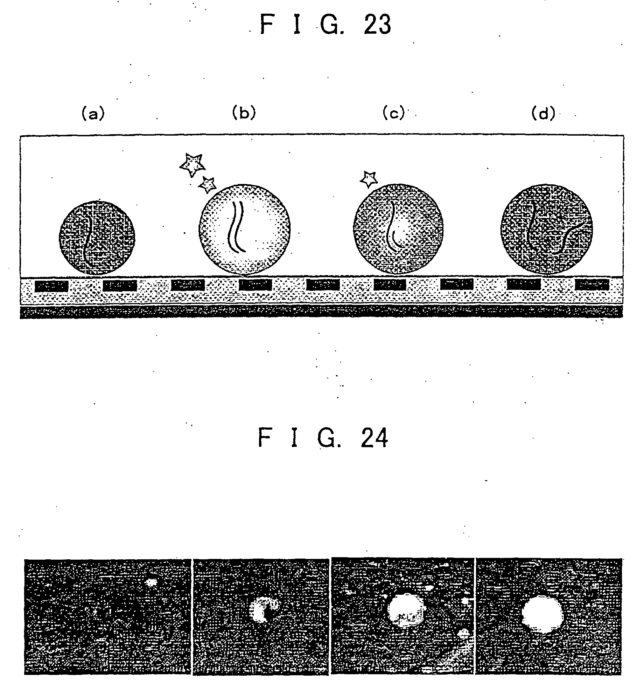

Fig. 23 illustrates a mechanism of fluorescence emission as a result of combination. Fig. 23 (a) illustrates a single-stranded DNA having a known base sequence; Figs. 23(b), 23(c) and 23(d) show the cases where the unknown single-stranded DNA perfectly matches, partially matches, and does not match, with the single-stranded DNA having a known base sequence, respectively.

With reference to Fig. 23, intense fluorescence is emitted only in the case where the unknown single-stranded DNA perfectly matches with the single-stranded DNA having a known base sequence [Fig. 23(b)].

The system according to the present invention serves as a DNA chip in which droplets each containing single-stranded DNA in a liquid are moved and reacted by the action of electrostatic force, to thereby detect genes.

Droplets containing different known single-stranded DNAs and a fluorescence reagent are arranged in an array in a liquid inert to DNAs. Droplets containing an unknown single-stranded DNA are arranged in the liquid and are electrostatically moved, are combined with the droplets containing the known single-stranded DNAs, respectively, thus carrying out hybridization. If the sample is complementary with the known single-stranded DNA, they undergo complete hybridization, and the fluorescence reagent is thus intercalated to emit fluorescence. Otherwise, the fluorescence reagent is not intercalated and does not emit fluorescence (Fig. 23(d)).

The base sequence of a gene is generally identified by a primer comprising about 20 bases. By using single-stranded DNA having 20 bases, it can be determined whether or not a gene has a base sequence as specified by the single-stranded DNA, as in regular DNA chips.

According to the present invention, a sample to be assayed is used in the form of droplets, and only a trace amount of the sample is required. In addition, it takes a very short time to perform hybridization, and the assay using the DNA chip according to the present invention exhibits many advantages as compared with conventional DNA chips.

Fig. 24 shows, from the left side, fluorescence intensities in the cases where a fluorescence reagent is present alone, where single-stranded DNA does not match with the fluorescence reagent, where DNA partially matches with the fluorescence reagent, and where DNA perfectly matches with the fluorescence reagent, respectively.

Fig. 24 shows that intense fluorescence is emitted only in the case where the sequences perfectly match with each other, and that slight fluorescence is emitted in the case where they match with each other only partially. The DNA sequence used herein comprises 20 bases, i.e., AGGGG ACTTT CCTGA CGTGT. The term "partially matches" as used herein means that only ten of the twenty bases match between two sequences.

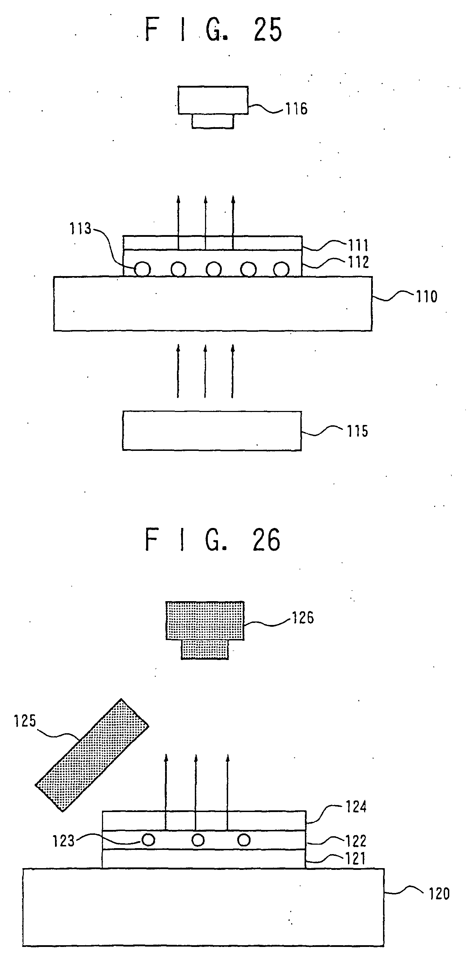

Fig. 25 is a schematic diagram of an image pickup device for determining the fluorescence intensities emitted upon hybridization as shown in Fig. 24.

Fig. 25 shows a base 110, an electrostatic transportation electrode substrate 111, a liquid layer 112, DNA-containing droplets 113, an ultraviolet ray source 115 for applying ultraviolet rays from below, and a camera (image pickup device) 116.

Fig. 26 is a schematic diagram of an image pickup device for determining the fluorescence intensities emitted upon hybridization as yet another embodiment of the present invention.

Fig. 26 shows a base 120, an electrostatic transportation electrode substrate 121, a liquid layer 122, DNA-containing droplets 123, a glass cover 124, an ultraviolet ray source 125 for applying ultraviolet rays diagonally from above, and a camera (image pickup device) 126. The glass cover 124 is not required in some cases.

Thus, the hybridization of DNA-containing droplets in the liquid layer can be observed as an image taken by the camera (image pickup device).

The present inventors filed patent applications on the handling of small liquid particles in an inert liquid as base technologies relating to the present invention (Japanese Patent Applications No. 2001-48096 and No. 2001-238624 under the title of "SMALL LIQUID PARTICLE HANDLING METHOD AND DEVICE THEREFOR").

The present invention is not specifically limited to the above-mentioned embodiments, and the invention is intended to cover and include various modifications and equivalent arrangements included within the spirit and scope of the invention.

As has been described in detail above, the present invention exhibits the following advantages.
(A) DNA-containing droplets as a biological sample can be accurately transported, the temperature of the droplets can be precisely controlled, and a primer can be reacted therewith in a simple and precise manner.
(B) The droplets are electrostatically transported and can thereby be moved two-dimensionally above electrodes. The PCR system does not require special micro-fluidic devices such as microchannels, microvalves and micropumps.
(C) Plural chemical reactions can be performed simultaneously or sequentially on a substrate, and the micro-droplets can be heated to a suitable temperature at a predetermined position by utilizing the electric conductivity of the micro-droplets and passing. an electric current therethrough.
(D) The temperature of the cell on the chip can be set at a predetermined temperature using a thermostatic heater.
(E) A large multiplicity of PCR can be efficiently performed sequentially or simultaneously in a flow system, by arranging electrodes and heating regions along the flow of the biological sample and performing individual PCR cycles sequentially at certain time intervals.
(F) The position of the biological sample can be accurately detected and controlled.
(G) The hybridization can be rapidly and easily detected.
(H) A sample to be assayed is used in the form of droplets and only a trace amount of the sample is required. In addition, it takes a very short time to perform hybridization, and the assay using the DNA chip according to the present invention exhibits many advantages as compared with conventional detection methods using DNA chips.

### Industrial Applicability

The present invention is suitable as a polymerase chain reaction (PCR) device and a hybridization device utilizing electrostatic transportation and is applicable to a wide variety of applications such as assay devices for biotechnological samples using a simple chip.

## Claims

1. A method for carrying out PCR utilizing electrostatic transportation, comprising the steps of:
(a) preparing a biological sample in a chemically inert liquid layer;
(b)electrostatically transporting the biological sample by the action of an electrostatic transportation plate with electrostatic transportation electrodes; and
(c) controlling temperatures at predetermined positions of the electrostatic transportation plate and carrying out polymerase chain reaction.

2. The method for carrying out PCR utilizing electrostatic transportation according to claim 1, wherein the biological sample comprises droplets containing DNA and primer.

3. The method for carrying out PCR utilizing electrostatic transportation according to claim 1, wherein the biological sample comprises droplets containing DNA, and droplets containing primer.

4. The method for carrying out PCR utilizing electrostatic transportation according to claim 1, further comprising arranging the electrodes in matrix form and electrostatically transporting the biological sample two-dimensionally.

5. The method for carrying out PCR utilizing electrostatic transportation according to claim 1, further comprising applying voltage to heating electrodes and passing an electric current through the droplets to thereby heat the droplets.

6. The method for carrying out PCR utilizing electrostatic transportation according to claim 5, wherein high-frequency alternating voltages are applied as the voltage.

7. The method for carrying out PCR utilizing electrostatic transportation according to claim 1, further comprising maintaining the chemically inert liquid layer at a first temperature with thermostatic heater.

8. The method for carrying out PCR utilizing .electrostatic transportation according to claim 1, further comprising heating the biological sample in multiple steps.

9. The method for carrying out PCR utilizing electrostatic transportation according to claim 1, further comprising arranging the electrodes and regions to be heated in a cascade and treating a plurality of the biological sample sequentially at specific time intervals.

10. A device for carrying out PCR utilizing electrostatic transportation, comprising:
(a) an electrostatic transportation plate with electrodes, the electrodes each having a water-repellent coating on a surface thereof;
(b) means for electrostatically transporting a biological sample by the action of the electrostatic transportation plate; and
(c) means for controlling the temperature of the biological sample at predetermined positions of the electrostatic transportation plate.

11. The device for carrying out PCR utilizing electrostatic transportation according to claim 10, wherein the electrodes are arranged in matrix form.

12. The device for carrying out PCR utilizing electrostatic transportation according to claim 10, further comprising a sheet-like heater layer for thermostatically heating the chemically inert liquid layer.

13. The device for carrying out PCR utilizing electrostatic transportation according to claim 10, wherein the means for heating the biological sample is a heating plate for applying voltage to the droplets.

14. The device for carrying out PCR utilizing electrostatic transportation according to claim 13, wherein the voltage is a high-frequency alternating voltage.

15. The device for carrying out PCR utilizing electrostatic transportation according to claim 10, wherein the means for heating the biological sample is a long heater layer.

16. A method for carrying out PCR utilizing electrostatic transportation, comprising the steps of:
(a) preparing droplets containing DNA and primer in a chemically inert liquid layer;
(b) electrostatically transporting the droplets containing DNA and primer by the action of an electrostatic transportation plate with electrostatic transportation electrodes;
(c) heating and thermally denaturing the DNA at a first position of the electrostatic transportation plate;
(d) annealing primer to the DNA, the primer being capable of reacting with the DNA; and
(e) elongating the annealed DNA.

17. A device for carrying out PCR utilizing electrostatic transportation, comprising:
(a) an electrostatic transportation plate with electrodes, the electrodes each having a water-repellent coating on a surface thereof;
(b) a chemically inert liquid layer carrying droplets containing DNA and primer;
(c) means for heating the droplets containing DNA and primer at predetermined positions;
(d) means for electrostatically transporting the droplets containing DNA and primer by the action of the electrostatic transportation plate;
(e) means for heating and thermally denaturing the DNA at a first position of the electrostatic transportation plate;
(f) means for annealing primer to the DNA, the primer being capable of reacting with the DNA; and
(g) means for elongating the annealed DNA.

18. The device for carrying out PCR utilizing electrostatic transportation according to claim 17, wherein the electrodes are arranged in matrix form.

19. The method for carrying out PCR utilizing electrostatic transportation according to claim 1 or 16, further comprising detecting and controlling the position of the biological sample.

20. The device for carrying out PCR utilizing electrostatic transportation according to claim 10 or 17, further comprising means including a computer, an image pickup device being connected to the computer, and a controller being connected to the computer, wherein the means is so configured as to monitor the motion of the biological sample with the image pickup device and to control the position of the biological sample by the controller through the computer based on the monitoring.

21. A method for carrying out hybridization utilizing electrostatic transportation, comprising the steps of covering an electrostatic transportation electrode substrate with a chemically inert liquid, arranging an array of droplets each containing a known single-stranded DNA and fluorescence reagent therein, electrostatically transporting droplets containing an unknown single-stranded DNA sample, combining the sample droplets with the respective droplets containing the known single-stranded DNA and the fluorescence reagent to thereby carry out hybridization, and detecting the hybridization based on the fact that the fluorescence reagent is intercalated between the known and unknown single-stranded DNAs when the two DNAs match with each other, and that fluorescence is emitted at a varying intensity depending on the degree of match between the two DNAs.

22. A device for carrying out hybridization utilizing electrostatic transportation, comprising:
(a) an electrostatic transportation electrode substrate being filled with a chemically inert liquid layer;
(b) droplets being arranged in an array on the electrostatic transportation electrode substrate and each comprising known single-stranded DNA and fluorescence reagent;
(c) droplets containing unknown single-stranded DNA sample to be transported by the action of electrodes of the electrostatic transportation electrode substrate; and
(d) means for treating the droplets containing the unknown single-stranded DNA sample with each of the droplets containing the known single-stranded DNAs and the fluorescence reagent and detecting the hybridization between the two DNAs.
